# EUROPÄISCHE PATENTSCHRIFT

(11) **EP 1 645 869 B1**
(45) Veröffentlichungstag und Bekanntmachung des Hinweises auf die Patenterteilung: **18.07.2007**
(21) Anmeldenummer: 05021996.3
(22) Anmeldetag: 23.04.2003
(51) Int. Cl.: G01N 25/14, G01N 33/22

(54) **Vorrichtung zur Aufnahme der Siedekurve von Flüssigkeiten**
Device for recording the boiling-point curve of liquids
Dispositif pour relever la courbe des points d'ébullition de liquides

(30) Priorität: 24.04.2002 AT 2702002
(43) Veröffentlichungstag der Anmeldung: 12.04.2006
(62) Teilanmeldung aus: 03717025.5
(73) Patentinhaber: Grabner Instruments Messtechnik GmbH, 1220 Wien (AT)
(72) Erfinder: Burian, Matthias, 2231 Strasshof (AT); Aschauer, Roland, 1220 Wien (AT)
(74) Vertreter: Haffner, Thomas M.

(56) Entgegenhaltungen:
- DE-A- 3 039 487
- FR-A- 2 815 413
- US-A- 3 364 731
- US-A- 3 399 116
- US-A- 4 250 739
- US-A- 4 528 635
- PATENT ABSTRACTS OF JAPAN Bd. 012, Nr. 175 (P-707), 25. Mai 1988 (1988-05-25) -& JP 62 285052 A (IDEMITSU KOSAN CO LTD), 10. Dezember 1987 (1987-12-10) & DATABASE WPI Section Ch, Week 198804 Derwent Publications Ltd., London, GB; Class H04, AN 1988-024755 & JP 62 285052 A (IDEMITSU KOSAN CO LTD) 10. Dezember 1987 (1987-12-10)
- PATENT ABSTRACTS OF JAPAN Bd. 1996, Nr. 10, 31. Oktober 1996 (1996-10-31) & JP 08 159993 A (TANAKA KAGAKU KIKI SEISAKU KK), 21. Juni 1996 (1996-06-21)

## Beschreibung

Die Erfindung bezieht sich auf eine Vorrichtung zur Aufnahme der Siedekurve von Flüssigkeiten, insbesondere Erdölprodukten und/oder Lösungsmitteln, wobei eine Probemenge der zu analysierenden Flüssigkeit verdampft und anschließend kondensiert wird und die Dampftemperatur und die jeweils verdampfte Menge der Flüssigkeit überwacht wird, mit einer über eine Leitung mit einer Probenschale verbundenen Fülleinrichtung zum Einfüllen einer Probemenge in eine Probenschale, wobei die Probenschale mit einer Destilliereinrichtung verbunden ist und die Fülleinrichtung und ein Kondenser zum Auffangen des Kondensats mit einer Temperaturregeleinrichtung zur Einstellung eines definierten, vorzugsweise gleichen Temperaturniveaus verbunden sind, wobei der Kondenser einen axialen Bereich mit verringertem Durchmesser aus einem für Licht, insbesondere Infrarotlicht durchsichtigen Material, insbesondere Glas, aufweist, an welchen ein einen axial beweglichen Kolben aufnehmender Bereich mit größerer lichter Weite anschließt und der Destillationsrückstand der Probenmenge durch Wägen bestimmbar ist.

Die Destillationseigenschaften von Flüssigkeiten und insbesondere von Erdölprodukten, wie beispielsweise von Treibstoff, sind in zahlreichen Produktspezifikationen festgelegt. Im Fall von Treibstoffen soll auf diese Weise sichergestellt werden, dass Motoren in den üblicherweise vorkommenden Leistungs- und Temperaturbereichen mit einem entsprechenden Treibstoff arbeiten. Weltweit anerkannt gibt es in diesem Zusammenhang ein Standardverfahren der Siedeanalyse für Erdölprodukte, das von ASTM und ISO anerkannt ist. Bei diesem Verfahren werden 100 ml der zu untersuchenden Flüssigkeit in einem beheizten Destillierkolben aus Glas destilliert und in einem gekühlten Kondenserrohr kondensiert. Das Kondensat wird in einem graduierten Glaszylinder aufgefangen. Zwischen Siedebeginn und Siedeende werden Dampftemperatur und bisher kondensiertes Volumen bestimmt (Siedekurve). Das Volumen des Destillationsrückstandes, der nach Abschalten der Heizung im Destillierkolben verbleibt, wird mit einem Messzylinder bestimmt, und die Siedekurve wird entsprechend korrigiert.

Diese Methode wurde um 1920 entwickelt. Die Regelung der Heizung, das Ablesen der Dampftemperatur und die Bestimmung des korrespondierenden Kondensatvolumens wurde dabei vom Bediener der Apparatur durchgeführt.

In der US 4,528,635 wird eine automatische Destillationsapparatur beschrieben. Diese Apparatur verwendet die gleichen Destillierkolben, Kondenser und Glaszylinder wie die ursprüngliche manuelle Methode. In der Apparatur wurde eine Vorrichtung zur Bestimmung des kondensierten Volumens eingebaut, sowie ein Microprozessor, der die Heizung und Kühlung kontrolliert sowie automatisch Messdaten aufnimmt. Grundsätzlich jedoch blieb die Methode unverändert.

Diese Standardmethode hat eine Reihe von Nachteilen für die Anwender. Das Probenvolumen ist mit 100 ml hoch, Probe und Destillationsrückstand müssen händisch abgemessen und eingefüllt werden, die Glaskolben und -zylinder sind zerbrechlich und müssen nach jeder Destillation gereinigt werden, die Messzeit ist hoch, und die Apparatur ist großvolumig und kann nur im Labor verwendet werden.

Vorrichtungen der eingangs genannten Art sind in unterschiedlichen Ausbildungen bekannt und beispielsweise auch der DE 3039487, der US 3,364,731 und der JP 62285052 oder der US 4,250,735 zu entnehmen.

Die DE 3039487 offenbart ein kontinuierlich ablaufendes Kurzweg-Destillationsverfahren zur Aufnahme der Siedekurve und eine entsprechende Vorrichtung für hochsiedende Flüssigkeiten, insbesondere Erdölprodukte. Aus einem Vorratsgefäß wird die Probemenge über eine als Zahnradpumpe ausgebildete Dosierpumpe einer beheizten Fläche zugeführt und auf dieser als dünner, ständig bewegter Film ausgebreitet. Der Dampf schlägt sich an einem im kurzen Abstand davon angeordneten Kondensator nieder. Das Destillat steht am Destillatauslauf, die unveränderten Rückstandsanteile am Rückstandsauslauf zur Verfügung. Das Vorratsgefäß, die Dosierpumpe, der Zuführung der Probenmenge dienenden Bauteile, die Kurzwegdestillationsanlage, der Destillatauslauf und der Rückstandsauslauf werden unabhängig voneinander beheizt und wahlweise auf unterschiedliche Temperaturen eingestellt. Destillats- und Rückstandsfraktionen werden gewogen.

Die US 3,364,731 zeigt und beschreibt ein Destillationsverfahren, bei welchem die Destillationsrate überwacht wird, wobei ein beweglicher Kolben im Aufnahmegefäß für das Destillat zur Konstanthaltung des Flüssigkeitsspiegels bewegt wird und aus der Bewegungsgeschwindigkeit des Kolbens auf die Destillationsrate geschlossen wird.

Neben der erfindungsgemäß angestrebten Miniaturisierung und Automatisierung zielt die Erfindung gleichzeitig darauf ab, bei einer Verkürzung der Messzeit und gleichzeitiger Verringerung der erforderlichen Probemenge Resultate zu liefern, welche der ASTM-Standardmethode äquivalent sind und auf Grund der weitestgehenden Automatisierung ein höheres Maß an Genauigkeit und Reproduzierbarkeit der Messergebnisse liefern. Insbesondere zielt die Erfindung darauf ab eine vollautomatische Führung des Verfahrens zu ermöglichen und die gesamte für die Durchführung des Verfahrens erforderliche Einrichtung in ihren Baumaßen so weit zu reduzieren, dass sie in einem konventionellen Kraftfahrzeug, beispielsweise im Kofferraum, untergebracht werden und vom Bordnetz eines derartigen Kraftfahrzeuges mit Energie versorgt werden kann.

Zur Lösung dieser Aufgaben besteht die erfindungsgemäße Vorrichtung im Wesentlichen in der Kombination der eingangs angeführten Merkmale aus einem für Licht, insbesondere Infrarotlicht durchsichtigen Material, insbesondere Glas, aufweist, an welchen ein einen axial beweglichen Kolben aufnehmender Bereich mit größerer lichter Weite anschließt. Ein derartiger beispielsweise aus Glasröhrchen ausgebildeter Abschnitt kann entsprechend geringen lichten Querschnitt aufweisen, sodass bereits geringe Volumsänderungen eine entsprechende Veränderung des Flüssigkeitsspiegels, beziehungsweise des Meniskus, zu Folge haben. Um dennoch eine entsprechend größere Menge des Kondensats aufnehmen zu können, schließt an ein derartiges Glasröhrchen der axial bewegliche Kolben in einem Zylinder mit entsprechend größerer lichter Weite an, wobei die Messung in einfacher Weise als Messung des Kolbenstellweges erfolgen kann, über welchen auf das entsprechende Volumen geschlossen werden kann. Zu diesem Zweck ist mit Vorteil die Ausbildung so getroffen, dass der axial bewegliche Kolben mit einem Stellantrieb, insbesondere einem Schrittmotor oder einem Getriebemotor mit einem Drehstellungsgeber verbunden ist, welcher in Abhängigkeit von den Signalen eines optischen Detektors antreibbar ist, wobei vorzugsweise der optische Signalgeber im Bereich des axialen Bereiches aus für Licht durchsichtigem Material angeordnet ist und zur Detektion des Meniskus der kondensierten Flüssigkeit ausgebildet ist und dass der Stellantrieb des Kolbens zur Korrektur der Position des Meniskus antreibbar ist.

Es gelingt somit auch bei kleinbauenden Einrichtungen durch entsprechende Thermostatierung, die entsprechenden Fehler gering zu halten und die Präzision der Ergebnisse zu steigern, sodass insgesamt eine portable Vorrichtung realisierbar wird. Dadurch, dass nun, wie es einer bevorzugten Weiterbildung entspricht, die Probenschale auf einer verfahrbaren Unterlage angeordnet ist, welche mit einem Stellantrieb verbunden ist, wird die Möglichkeit geschaffen, eine kostengünstige Alternative für Glasgefäße zu verwenden, und insbesondere die Möglichkeit geschaffen, eine derartige Probenschale nach ihrer einmaligen Verwendung auch zu verwerfen, da sie einen hinreichend billigen Bauteil darstellt. Die Probenschale kann hierbei mittels des Stellantriebes gegen die Destillationseinrichtung angestellt werden und durch Anpressen mit der Destillationseinrichtung entsprechend gasdicht verbunden werden, wobei gleichzeitig die Probenschale entsprechend beheizt werden kann, um eine Destillation zu ermöglich. Weiters erlaubt es der verfahrbare Stellantrieb, die Probenschale am Siedeende wieder abzuziehen und einer Wägeeinrichtung zuzuführen, um die entsprechende Bestimmung des Destillationsrückstandes mit hoher Präzision zu ermöglichen .

Im Sinne einer kleinbauenden portablen Einrichtung, welche auch mit einfachem Batteriebetrieb mit Energie versorgt werden kann, ist die Ausbildung mit Vorteil so getroffen, dass die Temperaturregeleinrichtung als elektrische Kühl- und/oder Heizeinrichtung, insbesondere unter Verwendung von Peltierelementen, ausgebildet ist. Derartige Peltierelemente werden in der Regel so gepolt, dass sie als Kühleinrichtung zum Einsatz gelangen. Durch Umpolung kann aber bei entsprechend ungünstigen Witterungsbedingungen auch eine entsprechend höhere Temperatur gewählt werden, um zu mit Standardtests vergleichbaren Ergebnissen zu gelangen.

Eine vollständig autonome und tragbare Vorrichtung ergibt sich dann, wenn die Fülleinrichtung und die Destillationseinrichtung in einem gemeinsamen, tragbaren Gehäuse angeordnet sind.

Die entsprechende Kompensation beziehungsweise Korrektur der volumetrischen Messergebnisse kann in einfacher Weise dadurch gewährleistet werden, dass die Fülleinrichtung und der Kondenser Temperatursensoren aufweisen.

Der Stellantrieb für die Probenschale kann in einfacher Weise als Getriebemotor ausgebildet sein, wodurch auch ein entsprechender Anpressdruck beim Anheben der Probenschale gewährleistet werden kann. Zur Erzielung eines dichten Abschlusses zwischen Probenschale und Destillierkolonne ist die Ausbildung mit Vorteil so getroffen, dass der Rand der Probenschale und der Rand der Anschlussöffnung der Destillierkolonne konisch, hohlkonisch oder ballig zur gasdichten Verbindung der Probenschale mit der Destilliereinrichtung unter Anwendung des vom Stellantrieb erzeugten Anpressdruckes ausgebildet sind.

Um auch hier thermische Fehler, welche zu einer Verzerrungen der Messwerte führen könnten, mit Sicherheit zu vermeiden, ist die Ausbildung mit Vorteil so getroffen, dass die Destillierkolonne von einer Isolation umgeben ist.

Wie bereits erwähnt, kann in besonders einfacher Weise die Destilliereinrichtung aus Edelstahl, Messing oder Titan und die Probenschale aus Metall, vorzugsweise Aluminium oder Kupfer bestehen. Eine derartige Ausbildung zeichnet sich durch entsprechend kostengünstige Gestaltung der Probenschale bei gleichzeitig einfacher homogener Temperaturverteilung aus.

Die ASTM-Standardmethode ist auf eine Siedeanalyse unter atmosphärischem Druck ausgelegt. Mit Vorteil ist daher die Ausbildung so getroffen, dass ein Drucksensor, insbesondere ein piezoresistiver Drucksensor zur Bestimmung des Luftdruckes vorgesehen ist und dass die Destillationseinrichtung im Bereich des Kondensers in offener Verbindung zur Atmosphäre ausgebildet ist, sodass eine entsprechend kompensierende Rechnung auf der Basis der Druckmesswerte jederzeit gelingt. Eine vollständige Erfassung aller Messwerte im gleichen Gerät und insbesondere auch eine gewichtsmäßige Erfassung des in der Probenschale enthaltenen Destillationsrückstandes gelingt mit Vorteil dann, wenn die Ausbildung so getroffen ist, dass im Gehäuse eine Waage angeordnet ist.

Die vollautomatische Durchführung des im Rahmen einer derartigen Vorrichtung durchführbaren Verfahrens gelingt in besonders einfacher Weise dann, wenn die Messwerte über Leitungen einem Mikroprozessor zugeführt sind und dass eine Anzeige bzw. Ausgabeeinrichtung für die Messergebnisse vorgesehen ist.

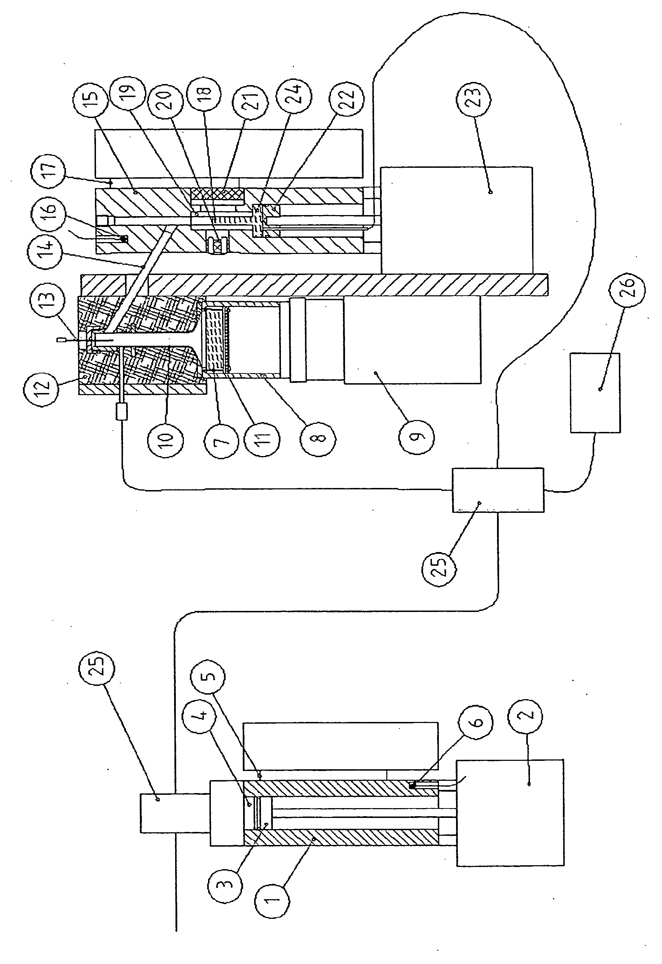

Die Erfindung wird nachfolgend anhand eines in der Zeichnung schematisch dargestellten Ausführungsbeispieles der erfindungsgemäßen Vorrichtung sowie einer Verfahrensbeschreibung näher erläutert.

Die Fülleinrichtung 1 besteht aus einem mit einem Schrittmotor oder Getriebemotor mit Drehgeber 2 angetriebenen Kolben 3, der in einer vernickelten Aluminiumkammer 4 bewegt wird und mit einem O-Ring gedichtet wird. Volumsauflösung ist besser als 1_. Die Temperatur der Fülleinrichtung 1 wird mit einem Peltierelement 5 reguliert und mit einem Temperatursensor 6, vorzugsweise einem Pt-100-Widerstandssensor, gemessen.

Die Probenschale 7 besteht aus tiefgezogenem Aluminium. Dadurch ist sie billig und wird nach der Messung weggeworfen, sodass eine aufwendige Reinigung entfällt. Die Probenschale 7 wird über einen Träger 8, der mit einem Getriebemotor 9 bewegt werden kann, gegen die Edelstahl-Destillationssäule 10 gedrückt. Im Träger 8 ist auch die elektrische Heizung 11 integriert. Auf die Destillationssäule 10 ist ein abgeschrägter Ring gedreht, an den die Probenschale gedrückt wird. Dadurch entsteht eine luftdichte Messkammer. Die Destillationssäule 10 ist außen mit Mineralwolle 12 isoliert, sodass Unterschiede der Umgebungstemperatur keinen Einfluss auf die Dampftemperatur haben können.

Die Dampftemperatur wird mit dem Thermoelement 13 bestimmt. Seitlich an der Destillationssäule 10 befindet sich ein Auslass 14, der in den Kondenser 15 mündet. Der Kondenser 15 besteht aus Aluminium und ist oben offen, sodass die Destillation unter dem vorherrschenden Luftdruck stattfindet. Die Kondensertemperatur wird mit einem Temperatursensor 16, vorzugsweise einem Pt-100-Widerstandsthermometer, gemessen und mit Hilfe eines Peltierele-mentes 17 konstant gehalten.

Im Kondenser eingebaut ist ein System 18 zur Volumsdetektion. Dieses System besteht aus einem Glasrohr 19, das in den Kondenser eingebaut ist und dessen Temperatur daher gleich der des Kondensers 15 ist. Zwei LEDs 20 beleuchten das Glasrohr 19 mit einem etwa parallelen Strahl. Ein optischer Zeilendetektor 21 (vorzugsweise ein Photodiodenarray) ist so angebracht, dass die Dioden im Brennpunkt der Zylinderlinse sitzen, die das Glasrohr 19 bildet, wenn es mit Probe gefüllt ist. Ist das Glasrohr 19 nun bis etwa zur Hälfte mit Flüssigkeit gefüllt, wird der Lichtstrahl der LEDs 20 in dem Bereich, in dem sich die Flüssigkeit befindet, auf den Zeilendetektor 21 fokussiert. Oberhalb der Grenzfläche Flüssigkeit-Luft (Meniskus) ist diese Zy-linderlinsenwirkung nicht gegeben. Dadurch registriert der Detektor 21 unterhalb des Meniskus erhöhte Lichtintensität, während oberhalb des Meniskus die Intensität niedriger ist.

Steigt der Meniskus im Glasrohr 19, so verändert sich ebenso die Intensitätsverteilung am Zeilendetektor 21. Die Ortsauflösung für die Bestimmung der Meniskusposition ist besser als 0,6 mm.

Die Position des Meniskus kann durch den Kolben 22, der von einem Schrittmotor oder einem Getriebemotor mit Drehgeber 23 in einer zylindrischen Kammer 24 bewegt wird, verändert werden. Die Änderung der Höhe des Meniskus nach einer Bewegung des Kolbens 22 wird mit dem Zeilendetektor 21 bestimmt.

Wenn nun Dampf im Kondenser kondensiert, steigt der Meniskus im Glasrohr 19 an. Wenn dieser Anstieg einen gewissen Wert erreicht, wird der Kolben 22 um einen vorbestimmten Betrag nach unten bewegt, wodurch der Meniskus wieder sinkt. Auf diese Weise wird der Meniskus immer im Bereich des Zeilendetektors 21 gehalten.

Aus dem bekannten Innendurchmesser des Glasrohres 19 und der Veränderung der Meniskushöhe wird das Volumen der kondensierten Probe bestimmt. Die Volumsauflösung ist dabei besser als 10µ_ und beträgt mindestens 0,1% des Füllvolumens.

Zur Verteilung der Probe in der Apparatur dienen Ventile 25. Ein Abfallbehälter 26 nimmt verbrauchte Probe auf.

Alle Sensoren, Motoren, Peltierelemente, Ventile und die Heizung werden von einem Mikroprozessor gesteuert und überwacht, sodass der Messvorgang nach einem vorgegebenen Programm vollautomatisch ohne Eingreifen des Bedieners abläuft.

Die ganze Vorrichtung kann in ein tragbares Gehäuse eingebaut werden.

### Messvorgang:

Zu Beginn einer Messung wird eine frische Probeschale 7 auf den Träger 8 gelegt und dadurch mit Hilfe des Motors 9 gegen die Destillationssäule 10 gepresst. Die Fülleinrichtung 1 und der Kondenser 15 werden auf gleiche Temperatur geregelt. Dann wird Probe mit der Fülleinrichtung 1 angesaugt. Ein vorgegebenes Volumen, vorzugsweise 6 ml, wird in die Probenschale 7 dosiert. Ebenso wird ein kleines Volumen in das Volumsdetektionssystem 18 gefüllt, sodass sich der Meniskus im Glasrohr 19 im Detektionsbereich des Zeilendetektors 21 befindet.

Die Heizung 11 wird nun aufgedreht. Die Probe beginnt kurz darauf zu sieden und zu verdampfen. Die Temperatur des aufsteigenden Dampfes wird mit dem Thermoelement 13 kontinuierlich gemessen. Die Leistung der Heizung hängt von dieser Dampftemperatur ab und wird vom Mikroprozessor so geregelt, dass sie mit steigender Dampftemperatur zunimmt.

Der Dampf tritt durch den Auslass 14 in den Kondenser 15 über und kondensiert dort wieder. Das kondensierte Volumen wird laufend mit dem Volumsdetektionssystem 18 gemessen.

Wenn die Probe vollständig verdampft ist, wird kein Dampf mehr nachgeliefert, und die Temperatur des Thermoelements 13 sinkt. Dann wird die Heizung 11 abgedreht. Sobald die Temperatur unter einen Schwellwert sinkt, fährt der Träger 8 mit der Probenschale 7 nach unten, und die Probenschale kann herausgenommen, gewogen und danach weggeworfen werden. Die verbrauchte Probe im Kondenser 15 wird über das Füllsystem 1 abgesaugt und in den Abfallbehälter 26 gefüllt.

Das Gewicht der Probenschale mit Rückstand wird eingegeben. Daraus wird vom Mikroprozessor der Destillationsrückstand berechnet, und die Siedekurve wird entsprechend korrigiert. Die Messresultate können auf einem eingebauten Display angezeigt, ausgedruckt oder auf einen Computer überspielt werden.

Die gesamte Messzeit beträgt etwa die Hälfte der für das ASTM-Standardverfahren notwendigen Zeit.

## Patentansprüche

1. Vorrichtung zur Aufnahme der Siedekurve von Flüssigkeiten, insbesondere Erdölprodukten und/oder Lösungsmitteln, wobei eine Probemenge der zu analysierenden Flüssigkeit verdampft und anschließend kondensiert wird und die Dampftemperatur und die jeweils verdampfte Menge der Flüssigkeit überwacht wird, mit einer über eine Leitung mit einer Probenschale (7) verbundenen Fülleinrichtung (1) zum Einfüllen einer Probemenge in eine Probenschale (7), wobei die Probenschale (7) mit einer Destilliereinrichtung verbunden ist und die Fülleinrichtung (1) und ein Kondenser (15) zum Auffangen des Kondensats mit einer Temperaturregeleinrichtung zur Einstellung eines definierten, vorzugsweise gleichen Temperaturniveaus verbunden sind, wobei der Kondenser (15) einen axialen Bereich mit verringertem Durchmesser aus einem für Licht, insbesondere Infrarotlicht durchsichtigen Material, insbesondere Glas, aufweist, an welchen ein einen axial beweglichen Kolben (22) aufnehmender Bereich mit größerer lichter Weite anschließt und der Destillationsrückstand der Probenmenge durch Wägen bestimmbar ist.

2. Vorrichtung nach Anspruch 1, **dadurch gekennzeichnet, dass** der axial bewegliche Kolben (22) mit einem Stellantrieb, insbesondere einem Schrittmotor oder einem Getriebemotor mit einem Drehstellungsgeber (23) verbunden ist, welcher in Abhängigkeit von den Signalen eines optischen Detektors (21) antreibbar ist.

3. Vorrichtung nach Anspruch 1 oder 2, **dadurch gekennzeichnet, dass** ein optischer Signalgeber (20) im Bereich des axialen Bereiches aus für Licht durchsichtigem Material angeordnet ist und zur Detektion des Meniskus der kondensierten Flüssigkeit ausgebildet ist und dass der Stellantrieb des Kolbens (22) zur Korrektur der Position des Meniskus antreibbar ist.

4. Vorrichtung nach Anspruch 1, 2 oder 3, **dadurch gekennzeichnet, dass** die Probenschale (7) auf einer verfahrbaren Unterlage (8) angeordnet ist, welche mit einem Stellantrieb (9) verbunden ist, durch welchen der Rand der Probenschale (7) an den Rand der Anschlussöffnung einer an die Probenschale (7) anschließenden Destillierkolonne (10) gedrückt werden kann.

5. Vorrichtung nach einem der Ansprüche 1 bis 4, **dadurch gekennzeichnet, dass** die Temperaturregeleinrichtung als elektrische Kühl- und/oder Heizeinrichtung, insbesondere unter Verwendung von Peltierelementen (5,17), ausgebildet ist und dass die Fülleinrichtung (1) und die Destillationseinrichtung in einem gemeinsamen, tragbaren Gehäuse angeordnet sind.

6. Vorrichtung nach einem der Ansprüche 1 bis 5, **dadurch gekennzeichnet, dass** die Fülleinrichtung (1) und der Kondenser (15) Temperatursensoren (6,16) aufweisen.

7. Vorrichtung nach einem der Ansprüche 1 bis 6, **dadurch gekennzeichnet, dass** der Stellantrieb (9) für die Probenschale (7) als Getriebemotor ausgebildet ist.

8. Vorrichtung nach einem der Ansprüche 1 bis 7, **dadurch gekennzeichnet, dass** der Rand der Probenschale (7) und der Rand der Anschlussöffnung der Destillierkolonne (10) konisch, hohlkonisch oder ballig zur gasdichten Verbindung der Probenschale (7) mit der Destilliereinrichtung unter Anwendung des vom Stellantrieb (9) erzeugten Anpressdruckes ausgebildet sind.

9. Vorrichtung nach einem der Ansprüche 1 bis 8, **dadurch gekennzeichnet, dass** die Destillierkolonne (10) von einer Isolation (12) umgeben ist.

10. Vorrichtung nach einem der Ansprüche 1 bis 9, **dadurch gekennzeichnet, dass** die Destilliereinrichtung aus Edelstahl, Messing oder Titan und die Probenschale (7) aus Metall, vorzugsweise Aluminium oder Kupfer besteht.

11. Vorrichtung nach einem der Ansprüche 1 bis 10, **dadurch gekennzeichnet, dass** ein Drucksensor, insbesondere ein piezoresistiver Drucksensor zur Bestimmung des Luftdruckes vorgesehen ist und dass die Destillationseinrichtung im Bereich des Kondensers (15) in offener Verbindung zur Atmosphäre ausgebildet ist.

12. Vorrichtung nach einem der Ansprüche 1 bis 11, **dadurch gekennzeichnet, dass** im Gehäuse eine Waage angeordnet ist, welche zum Abwiegen des Destillationsrückstandes der Probemenge ausgebildet ist.

13. Vorrichtung nach einem der Ansprüche 1 bis 12, **dadurch gekennzeichnet, dass** die Messwerte über Leitungen einem Mikroprozessor zugeführt sind, welcher zur Auswertung und Berechnung von Messergebnissen ausgebildet ist, und dass eine Anzeige bzw. Ausgabeeinrichtung für die Messergebnisse vorgesehen ist.

## Claims

1. A device for recording the boiling curve of liquids, in particular petroleum products and/or solvents, in which a sample amount of the liquid to be analyzed is evaporated and subsequently condensed, wherein the vapour temperature and the respectively evaporated amount of liquid are monitored, comprising a filling means (1), for filling in a sample amount, which is connected with a duct leading to a sample dish (7), whereby the sample dish (7) is connected with a distillation apparatus and the filling means (1) and the condenser (15) for the collection of condensation are connected to a temperature controller for setting a defined, preferably identical, temperature level, whereby the condenser (15) comprises and axial zone with reduced diameter of a material transparent to light and, in particular infrared light, particularly glass, which is followed by a zone having a larger clear width adapted to receive an axially moveable piston (22) and the distillation residue of the sample amount can be measured by weighing.

2. A device according to claims 1, **characterized in that** the axially movable piston (22) is connected with an adjustment drive, particularly a stepper motor or geared motor including a rotary encoder (23), which is actuatable as a function of the signals transmitted by an optical detector (21).

3. A device according to claims 1 or 2, **characterized in that** an optical signal transmitter (20) is arranged in the region of the axial zone of light-transparent material and designed for the detection of the meniscus of the condensed liquid, and that the adjustment drive of the piston (22) is actuatable for the correction of the position of the meniscus.

4. A device according to claim 1, 2 or 3, **characterized in that** the sample dish (7) is arranged on a movable support (8), which is connected with an adjustment drive (9), by which the edge of the sample dish (7) can be pressed at the edge of the connection opening of a distillation column (10) that follows the sample dish (7).

5. A device according to any one of claims 1 to 4, **characterized in that** the temperature controller is designed as an electric cooler and/or heater using, in particular, Peltier elements (5, 17), and that the filling means (1) and the distillation apparatus are arranged in a common portable housing.

6. A device according to any one of claims 1 to 5, **characterized in that** temperature sensors (6, 16) are provided in both the filling means (1) and the condenser (15).

7. A device according to any one of claims 1 to 6, **characterized in that** the adjustment drive (9) for the sample dish (7) is designed as a geared motor.

8. A device according to any one of claims 1 to 7, **characterized in that** the edge of the sample dish (7) and the edge of the connection opening of the distillation column (10) are designed to be conical, hollow-conical or ball-shaped, so as to ensure the gas-tight connection of the sample dish (7) with the distillation apparatus while applying the pressing pressure created by the adjustment drive (9).

9. A device according to any one of claims 1 to 8, **characterized in that** the distillation column (10) is surrounded by an insulation means (12).

10. A device according to any one of claims 1 to 9, **characterized in that** the distillation apparatus is made of stainless steel, brass or titanium, and the sample dish (7) is made of metal, preferably aluminum or copper.

11. A device according to any one of claims 1 to 10, **characterized in that** a pressure sensor, particularly a piezoresistive pressure sensor, is provided for the determination of the air pressure, and that the distillation apparatus in the region of the condenser (15) is designed with an open connection to the atmosphere.

12. A device according to any one of claims 1 to 11, **characterized in that** a weighing means designed for the weighing of the distillation residue of the sample amount is arranged within the housing.

13. A device according to any one of claims 1 to 12, **characterized in that** the measuring values are fed via lines to a microprocessor designed for the evaluation and calculation of measuring results, and that a display or output means for the measuring results is provided.

## Revendications

1. Dispositif pour relever la courbe des points d'ébullition de liquides, en particulier de produits pétroliers et/ou de solvants, où un échantillon du liquide à analyser est évaporé puis condensé et la température de vapeur et la quantité de liquide évaporé dans chaque cas sont surveillées, avec un dispositif de remplissage (1) relié par un conduit à une coupelle à échantillon (7) pour l'introduction d'un échantillon dans une coupelle à échantillon (7), où la coupelle à échantillon (7) est reliée à un dispositif de distillation et le dispositif de remplissage (1) et un condenseur (15) pour recevoir le condensat sont reliés à un dispositif de réglage de la température pour établir un niveau de température défini, de préférence uniforme, où le condenseur (15) comporte un domaine axial de diamètre réduit constitué par un matériau perméable à la lumière, en particulier à la lumière infrarouge, en particulier le verre, auquel se raccorde un domaine de plus grand diamètre intérieur recevant un piston (22) déplaçable axialement, et le résidu de distillation de l'échantillon peut être déterminé par pesée.

2. Dispositif selon la revendication 1, **caractérisé en ce que** le piston (22) déplaçable axialement est relié à un servomoteur, en particulier un moteur pas à pas ou un moto-réducteur avec un transmetteur de position de rotation (23) qui peut être actionné en fonction des signaux d'un détecteur optique (21).

3. Dispositif selon la revendication 1 ou 2, **caractérisé en ce qu'**un transmetteur de signaux optiques (20) est disposé dans le domaine du domaine axial en matériau perméable à la lumière et est agencé pour la détection du ménisque du liquide condensé et **en ce que** le servomoteur du piston (22) peut être actionné pour corriger la position du ménisque.

4. Dispositif selon la revendication 1, 2 ou 3, **caractérisé en ce que** la coupelle à échantillon (7) est disposée sur une base (8) déplaçable qui est reliée à un servomoteur (9) par lequel le bord de la coupelle à échantillon (7) peut être pressé contre le bord de l'ouverture de raccordement d'une colonne de distillation (10) qui se raccorde à la coupelle à échantillon (7).

5. Dispositif selon l'une des revendications 1 à 4, **caractérisé en ce que** le dispositif de réglage de la température est agencé sous forme de dispositif électrique de refroidissement et/ou de chauffage, en particulier au moyen d'éléments Peltier (5, 17), et **en ce que** le dispositif de remplissage (1) et le dispositif de distillation sont disposés dans un boîtier portable commun.

6. Dispositif selon l'une des revendications 1 à 5, **caractérisé en ce que** le dispositif de remplissage (1) et le condenseur (15) comportent des capteurs de température (6, 16).

7. Dispositif selon l'une des revendications 1 à 6, **caractérisé en ce que** le servomoteur (9) pour la coupelle à échantillon (7) est agencé sous forme de moto-réducteur.

8. Dispositif selon l'une des revendications 1 à 7, **caractérisé en ce que** le bord de la coupelle à échantillon (7) et le bord de l'ouverture de raccordement de la colonne de distillation (10) sont agencés de manière conique, conique creuse ou bombée pour la liaison étanche aux gaz de la coupelle à échantillon (7) avec le dispositif de distillation au moyen de la pression d'appui produite par le servomoteur (9).

9. Dispositif selon l'une des revendications 1 à 8, **caractérisé en ce que** la colonne de distillation (10) est entourée par une isolation (12).

10. Dispositif selon l'une des revendications 1 à 9, **caractérisé en ce que** le dispositif de distillation consiste en acier spécial, laiton ou titane et la coupelle à échantillon (7) consiste en métal, de préférence en aluminium ou en cuivre.

11. Dispositif selon l'une des revendications 1 à 10, **caractérisé en ce qu'**un capteur de pression, en particulier un capteur de pression piézo-résistif pour déterminer la pression de l'air est prévu et **en ce que** le dispositif de distillation est agencé en liaison ouverte avec l'atmosphère dans le domaine du condenseur (15).

12. Dispositif selon l'une des revendications 1 à 11, **caractérisé en ce qu'**il est prévu dans le boîtier une balance qui est agencée pour peser le résidu de distillation de l'échantillon.

13. Dispositif selon l'une des revendications 1 à 12, **caractérisé en ce que** les valeurs mesurées sont transmises par des conduits à un microprocesseur qui est agencé pour l'évaluation et le calcul des résultats des mesures, et **en ce qu'**il est prévu un indicateur ou un dispositif d'affichage pour les résultats des mesures.
